# EUROPEAN PATENT APPLICATION

(11) **EP 4 623 967 A1**
(43) Date of publication of application: **01.10.2025**
(21) Application number: 24166774.0
(22) Date of filing: 27.03.2024
(51) Int. Cl.: A61M 11/00, A61M 15/00, A61M 16/00

(54) **INHALATION THERAPY DEVICE**

(71) Applicant: PARI Pharma GmbH, 82319 Starnberg (DE)
(72) Inventor: STOECKL, Carolin, 80331 München (DE); HOFFMANN, Tobias, 86938 Schondorf am Ammersee (DE); HEINE, Benjamin, 80687 München (DE); FILIP, Eric, 81547 München (DE); REINHART, Markus, 86919 Utting (DE); VOIGT, Andre, 81543 München (DE)
(74) Representative: Hoffmann Eitle

(57) **Abstract**

The present disclosure relates to an inhalation therapy device. The inhalation therapy device comprises a housing, an aerosol generator configured to generate an aerosol from a liquid, a flow path, a measurement passage connecting the flow path and a sensor, as well as a pressure-transmitting member. The flow path is defined in the housing and comprises a first opening to the outside of the housing at one end and a second opening to the outside of the housing at another end, so that at least an inhalation flow from the second opening to the first opening is generatable in the flow path upon inhalation of a patient at the first opening for entraining and delivering the generated aerosol. The sensor is positioned outside the flow path and is configured to detect a pressure in the flow path via the measurement passage. The pressure-transmitting member is suitable to transmit the pressure in the flow path to the sensor, wherein the pressure-transmitting member closes the measurement passage and is impermeable to liquids.

## Description

### Technical Field

The present disclosure relates to an inhalation therapy device for entraining and delivering a generated aerosol to a user or patient.

### Background Art

Inhalation therapy devices, also known as (inhalation) nebulizers or aerosol delivery devices, are widely used to deliver therapeutically effective amounts of pharmaceuticals, such as drugs and vaccines, in an aerosol form to users through their respiratory system. Inhalation therapy devices can also be used for diagnostic purposes using radioisotopes for lung challenge tests. For therapy, aerosol inhalation is the preferred root of administration for some pharmaceuticals, which can be intended for treatment of systemic or respiratory diseases.

To achieve the intended treatment, aerosol particles must be deposited in specific parts of the respiratory tract of the user's body, such as the lung. Different size particles tend to deposit in different parts of the respiratory system. It is commonly known in this field that particles having a MMAD (Mass Median Aerodynamic Diameter) of less than Spm or even less than 4µm and at least 1µm are required to allow efficient deposition in the intended part of the respiratory system.

The aerosols for therapeutic purposes are generated and delivered to a desired location within the user's body, especially its respiratory tract, with the inhalation therapy device. To do so, a fluid, particularly a liquid, i.e., a medicament, a drug, a vaccine, etc., to be aerosolized or nebulized is supplied to a reservoir for holding the fluid or liquid in the inhalation therapy device.

For aerosolizing or nebulizing the liquid in the reservoir, the inhalation therapy device comprises an aerosol generator.

Such an aerosol generator may, for example, comprise a membrane unit, which comprises an aerosol-generating membrane having a plurality of apertures and an actuator that is directly or indirectly, via a support plate supporting the aerosol-generating membrane, coupled to the same for vibrating the aerosol-generating membrane.

The fluid or liquid may be in contact with a first side of the aerosol-generating membrane via gravitational force. Alternatively or additionally, the fluid or liquid may be in contact with the first side of the aerosol-generating membrane via evacuation of a closed, flexible container. The fluid or liquid (i.e., the medicament) passes through the apertures from the first side and an aerosol is generated at a second side of the aerosol-generating membrane opposite to the first side of the aerosol-generating membrane upon vibrating the same.

The aerosolized or nebulized fluid or liquid, i.e., the aerosol, inside the inhalation therapy device is then supplied to the user's respiratory system within the bounds of an inhalation therapy upon inhalation of the user through the inhalation therapy device. Meaning, upon the application of a suction force by the user's breathing, especially his or her inhalation, through the inhalation therapy device the aerosol is at least partially transported (by the suction force) from the interior of the inhalation therapy device to the user's respiratory tract.

Alternatively or additionally, the aerosol inside the inhalation therapy device may be supplied to the user's respiratory system within the bounds of an inhalation therapy upon breathing of the user using a breathing assistant device, such as for example a CPAP (Continuous Positive Airway Pressure), a BiPAP (Bilevel Positive Airway Pressure), a ventilator, or a respirator.

Examples for conventional inhalation therapy devices having such a configuration are, for example, derivable from EP 1 227 856 B1, US 6,962,151 B1, or DE 199 53 317 C1. The membrane unit of such a disclosed inhalation therapy device comprises, for example, a cylindrical liquid reservoir container, which is delimited at one end face by an aerosol-generating membrane having the shape of a circular disc. A liquid disposed in the reservoir contacts the side of the aerosol-generating membrane facing the reservoir.

The aerosol-generating membrane disclosed, for example, in DE 199 53 317 C1 is connected (welded) to a substrate (support plate) and an oscillating generator, for example, a piezo crystal, which surrounds the aerosol-generating membrane in a circular manner and is connected (glued) to the substrate, such that the aerosol-generating membrane can be caused to oscillate by means of the oscillating generator and an electric drive circuit.

Thereby, the liquid applied to the aerosol-generating membrane on the first side of the same is conveyed through the apertures in the oscillating aerosol-generating membrane to the second side of the aerosol-generating membrane opposite to said first side to be emitted into a chamber as an aerosol. The disclosed inhalation therapy device further embodies a mouthpiece, or a mask connected to an outlet of the chamber and having an exhalation valve. Upon exhalation of a user, the exhaled flow will, thus, not or only to a limited extend enter the chamber and rather be expelled through the exhalation valve into the environment of the device.

The disclosed device continuously drives the piezo crystal throughout the (single) treatment time (single administration interval), in which substantially the entire liquid in the reservoir is aerosolized by the aerosol-generating membrane. During exhalation of a user, the generated aerosol is temporarily stored in the chamber forming an aerosol cloud (bolus), which upon inhalation is inhaled and transported to the user's respiratory tract for therapy. Therefore, the treatment time can be kept relatively short.

One potential drawback is, however, that with an increasing output rate of the aerosol generator (aerosol-generating membrane, piezo crystal, drive circuit, etc.) the amount of aerosol deposited on the inner walls of the device, which is known as a so-called rainout, particularly in the chamber, increases. This leads to an increased loss of administrable aerosol/pharmaceutical.

Other conventionally known inhalation therapy devices drive the piezo crystal only during inhalation and stop driving the piezo crystal during exhalation. Those inhalation therapy devices are often referred to as breath-triggered devices or breath-actuated devices. It has been found that such inhalation therapy devices often have the issue of a long therapy duration (long administration interval), which can be annoying to the user and, thereby, provoke a lack of user adherence, e.g., an interruption or a premature end of the therapy before the intended dose of fluid or liquid in the reservoir has been nebulized and delivered to the user's respiratory tract for therapy.

The conventional inhalation therapy devices described above are oftentimes operated in such a manner that the user merely inhales through the inhalation therapy device, i.e., an inhalation flow is generated through a flow path of the device, and the inhalation therapy device is removed from the user's mouth and/or nose upon exhalation. While both enables a resistance-free exhalation of the user, the overall treatment cannot be satisfactorily monitored, as no data, such as a flow parameter in a flow path of the device, can be obtained during the exhalation. Additionally, and in case of a continuous operation, generated aerosol gets lost without any therapeutic effect for the user.

On top of that, removing the inhalation therapy device with every exhalation negatively affects the user's natural respiration, i.e., his or her steady breathing cycle.

Hence, conventional systems were confronted with a need for an effective inhalation therapy device that enables a short overall therapy time (single administration interval), while rainout and/or loss of generated aerosol without any therapeutic effect for the user can be reduced, and the user is given the possibility to make a break during the therapy, i.e., during a single administration interval, without any loss of the medication.

To achieve the same, various control mechanisms for the aerosol generation inside the inhalation therapy device have been proposed. While activating (during inhalation) and deactivating (during exhalation) the driving of the piezo crystal - in general - ensures that losses of the aerosolized fluid or liquid containing a pharmaceutical can be reduced, turning the aerosol generation on and off, i.e., performing the triggering at the right time is rather difficult.

On the one hand, the aerosol generation must start at the latest upon the start of the user's inhalation to omit an unnecessary prolongation of the treatment time. On the other hand, the production of aerosol must be stopped early enough that, upon exhalation of the user through the inhalation device, it can be avoided that produced aerosol is expelled from the inhalation therapy device and the user's upper respiratory tract without any therapeutic application.

To achieve the same, the so-called "pre-on/pre-off" inhalation therapy devices have been considered by the Applicant. Therein, the aerosol production is started in advance of the actual inhalation of the user, such that a certain amount of aerosol is generated before the actual inhalation, i.e., a pre-inhalation aerosol production similar to the aerosol bolus discussed above is produced, and the drive of the piezo crystal is stopped before the exhalation starts, i.e., a preexhalation aerosol production stop is performed.

To achieve the same, all non-continuously operating inhalation therapy devices require a precise monitoring of the user's breathing pattern at least in the inhalation direction to adequately control the drive of the piezo crystal and, thereby, the aerosol production.

Accordingly, most conventional non-continuously operating inhalation therapy devices comprise sensors. Such sensors observe the flows through the inhalation therapy device and are, therefore, necessary to precisely trigger the aerosol generation.

The previous solutions are, however, confronted with a deterioration of the quality of the sensor signal during a single or several consecutive administration interval(s). This is due to a contamination of the sensor, for example, by dust, dirt, germs, bacteria, viruses, humidity, fluids, sputum, and/or any other microbiologic contaminations stemming, for example, from the exhalation of the user into the inhalation therapy device. This provokes an adulteration of the sensor measurement results, consequently, a malfunction of the inhalation therapy device, and, ultimately, a decreased success of therapy as well as an increased risk of microbiological contamination of the inhalation therapy device, which accordingly induces an increased cleaning effort.

### Summary of the Disclosure

In view of the aforesaid, it is an object of the present disclosure to provide an inhalation therapy device that increases the overall quality of drug delivery in a consistent and reliable manner.

This object is solved by means of an inhalation therapy device according to independent claim 1. Distinct embodiments are derivable from the dependent claims.

According to a first aspect of the present disclosure, an inhalation therapy device comprises a housing, an aerosol generator configured to generate an aerosol from a liquid, a flow path, a measurement passage, and a pressure-transmitting member.

The flow path is defined in the housing. The flow path comprises a first opening to the outside, i.e., the environment, of the housing (inhalation therapy device) at one end and a second opening to the outside, i.e., the environment, of the housing (inhalation therapy device) at another end. Accordingly, at least an inhalation flow from the second opening to the first opening is generatable in the flow path upon inhalation of a patient at the first opening for entraining and delivering the generated aerosol.

This flow from the second opening to the first opening upon inhalation of the user at the first opening may, hence, also be understood as a first flow direction, an inhalation flow direction, or simply as an inhalation direction of the air flow, preferably air and aerosol flow, through the inhalation therapy device. It goes without saying that the inhalation therapy device is not bound to be operated by ambient air only. The gas entering the flow path may be ambient air, as well as 100% oxygen, or a mixture of oxygen and ambient air.

The first opening may, for example, be understood as a mouthpiece, an inhalation mask, or the like, or at least a connection to such elements through which the user can apply a suction force to the flow path of the inhalation therapy device.

Alternatively, the device can also be applied to ventilated patients, wherein the inhalation therapy device is connected to a breathing assistant device, such as for example a CPAP (Continuous Positive Airway Pressure), a BiPAP (Bilevel Positive Airway Pressure), a ventilator, or a respirator for entraining and delivering the generated aerosol to the user's respiratory system.

Accordingly, to establish said flow through the flow path in the first flow direction, i.e., the inhalation flow direction, air must accordingly be able to enter the flow path at the second opening. Accordingly, the second opening at least acts as an entrance for air when an inhalation of the user at the first opening of the therapy device is performed to entrain and deliver the generated aerosol inside the inhalation therapy device. During an inhalation phase, the second opening may, thus, also be referred to as "inlet opening" and the first opening may, thus, also be referred to as "outlet opening".

It may equally be possible to generate a flow in the flow path in an opposite, second flow direction from the first opening to the second opening upon exhalation of the user at the first opening for expelling the air from the respiratory tract. The second flow direction may, thus, be interpreted as an exhalation flow direction or simply an exhalation direction of the (air) flow through the inhalation therapy device. During an exhalation phase, the second opening may, thus, also be referred to as "outlet opening" and the first opening may, thus, also be referred to as "inlet opening".

This enables that the inhalation therapy device can stay in contact with the user (for example, his or her mouth, and thereby his or her respiratory tract) during the whole therapy duration (or treatment time) of a single administration interval (one inhalation therapy application), and the inhalation and the exhalation can be performed through the flow path and the air flow through the inhalation therapy device.

The measurement passage connects the flow path and a sensor. That is, the provision of the measurement passage enables that the sensor is positioned outside the flow path, i.e., distant from the surface of the flow path defined in the housing.

The sensor is configured to detect a pressure in the flow path via the measurement passage. Said pressure measurement via the flow path and the measurement passage, i.e., said pressure measurement in the air pathway through the inhalation therapy device, makes it, among others, possible to generate information on the flow, particularly the volume-flow rate, the inhalation volume and/or the exhalation volume as well as on the optimum start and stop points for the aerosol generation via the aerosol generator upon inhalation (and exhalation) of the patient at the first opening.

Said pressure measurement in the air pathway through the inhalation therapy device furthermore allows to obtain/generate/calculate information, among others, on the breathing frequency, the minute-volume, the I/E-ratio, the flow-values above a certain threshold, the cumulative-volume above a certain threshold, the minimum and the maximum of the flow, etc.

The measurement passage may, hence, also be understood as a (measurement) channel connecting the flow path and the sensor and the arrangement of the sensor as being outside of the flow path may equally be construed as being arranged away from the flow path and/or as not being directly positioned in said flow path.

Accordingly, dynamic pressure effects that would negatively affect the measurement of the pressure in the flow path via the sensor can be reduced.

Sensors which are configured to detect a pressure in the flow path are also known as pressure sensors in the technical field given.

The pressure-transmitting member of the inhalation therapy device is a structural element that is suitable/configured to transmit the pressure, which is generatable by the user's inhalation (and exhalation) through the inhalation therapy device, in the flow path and the measurement passage to the sensor.

In this connection, the pressure-transmitting member closes the measurement passage. In particular, the pressure-transmitting member closes the measurement passage to the flow path.

The pressure-transmitting member is thereby constituted in such a manner that it is impermeable to liquids.

Said constitution as being able/configured to transmit pressure is to be understood in such a manner that its shape and form enable to transmit and forward the aerosol/gas pressure through the inhalation therapy device to the pressure sensor without drastic losses that would deteriorate and/or adulterate the detected pressure values via the sensor. In other words, the pressure can be transmitted through the pressure-transmitting member, whereas the sensor "behind" the pressure-transmitting member can be protected from liquids of any kind, as the pressure-transmitting member closes the measurement passage. The term "behind" is in this regard to be understood as being arranged away from the flow path through the housing of the inhalation therapy device. Therefore, the pressure-transmitting member completely seals the measurement passage in a way that it is impermeable to liquids and, hence, acts as a hygienic barrier between the flow path and the sensor. As such, liquids cannot pass the pressure-transmitting member arranged in the measurement passage or at the interconnection of the flow path and the measurement passage.

As such, it is the key idea of the present disclosure to provide a sealing element, i.e., a member, in the measurement passage between the flow path and the sensor that is capable to transmit pressure generated by the inhalation (and exhalation) through the flow path, while being impermeable (at least) to liquids to protect the sensor and to thereby improve and maintain the quality of pressure detection. At the same time, it is possible to establish a hygienic protection for the user, which allows a cleaning and disinfection of the inhalation therapy device with liquids according to the instruction for use, such that a constant quality of pressure detection with the sensor at a high hygienic level is achievable.

While the pressure-transmitting member, thus, allows to transmit the pressure in the flow path to the sensor, it, at the same time, also enables to improve the measurement quality of said pressure sensor and thereby to improve the control of the inhalation therapy device. This is particularly the case, as the measurement surface of the pressure sensor can be protected from any liquids that would (negatively) affect the measurement of the sensor itself, which would ultimately affect the control of the inhalation therapy device, namely the aerosol generation via the aerosol generator. Furthermore, the interplay of the measurement passage allowing that the sensor is positioned outside and, thus, not directly in the flow path together with said pressure-transmitting member enables that dynamic pressure effects on the pressure detection are reduced or omitted, and the pressure sensor is protected from environmental influences, such as droplets of the aerosol, aerosol particles, sputum, any fluid or liquid from the (fluid) reservoir, dust, dirt, impurities, contaminants, germs, bacteria, viruses, humidity, sputum, and/or other microbiologic contaminations, etc.

Accordingly, the generatable flow through the flow path and the pressure detection can be functionally decoupled and, accordingly, the overall quality of drug delivery when using the inhalation therapy device can be increased in a consistent and reliable manner. On top of that, the freedom of constructional design can also be increased, as the sensor can be arranged distant from the flow path inside the housing of the inhalation therapy device.

Preferably, at least a surface of the pressure-transmitting member being oriented away from the sensor, optionally the entire pressure-transmitting member, comprises hydrophobic properties. These hydrophobic properties allow to avoid the deposition of the above-listed contaminations and, thereby, to achieve an improved quality of pressure detection over a long period of time.

It may, on top of that, also be possible that the pressure-transmitting member comprises not only hydrophobic properties but also lipophobic properties or other properties that avoid the deposition of said contaminations. This can, for example, be achieved by way of coatings or surface structures.

Accordingly, a contamination of the pressure-transmitting member, for example, by germs, bacteria, viruses, humidity, fluids, sputum and/or other microbiologic contaminations stemming, for example, from the exhalation of the user into the inhalation therapy device or from the suction of ambient air into the inhalation therapy device, and, thus, an adulteration of the pressure detection quality can be prevented.

According to a further aspect of the present disclosure, the housing comprises a nebulizer housing and a controller housing. The nebulizer housing defines the flow path and accommodates the aerosol generator. The nebulizer housing is detachable from the controller housing. The controller housing accommodates a controller configured to control the aerosol generator and the sensor.

A part of the measurement passage is formed in the nebulizer housing and a part of the measurement passage is formed in the controller housing. In this connection, the respective parts of the measurement passage are connected in a gas tight manner in the use state of the inhalation therapy device to ensure pressure transmission from the flow path through the entire measurement passage to the sensor.

The pressure-transmitting member closes the part of the measurement passage in the controller housing or the part of the measurement passage in the nebulizer housing.

The possibility to detach the nebulizer housing from the controller housing enables a facilitated cleaning, a facilitated storage and/or an exchange of the nebulizer housing and the controller housing, and/or facilitated disinfection of all components. It is, for example, possible to rinse all elements contained in the nebulizer housing, particularly the front and back of the aerosol generator, thoroughly under running tap water. Furthermore, it is possible to disinfect the nebulizer housing by way of using a thermal disinfector with an operation time of at least 6 minutes, or by way of using boiling water for approximately 15 minutes.

While the nebulizer housing defining the flow path is to be cleaned after every administration interval or at least every day, the controller housing, which does not come into direct contact with the actual aerosol or other contaminations during therapy and rather contains the major electric components for operating the inhalation therapy device, needs to be cleaned and/or disinfected after several consecutive administration interval(s) only, for example with a clean, lint-free, damp cloth. As such, a functional separation can be achieved, as the sensitive electronic parts in the controller housing are arranged separated from the actual flow path and the aerosol generation in the nebulizer housing.

When the nebulizer housing and the controller housing are in a non-detached state, i.e., when they are interconnected and, accordingly, in an attached state, these parts of the housing establish a sufficiently gas tight connection. This connection allows that pressure entering the flow path can be transported between the nebulizer housing and the controller housing, particularly between the part of the measurement passage in the controller housing and the part of the measurement passage in the nebulizer housing as well as via the pressure-transmitting membrane towards the sensor being arranged in the controller housing. This is obtained due to a gas tight connection of the controller housing and the nebulizer housing when the inhalation therapy device is in use. This may, for example be achieved by a form-fit connection of the controller housing and the nebulizer housing in an attached state and/or by the support of sealing members such as an O-ring, one or multiple sealing lip(s), etc.

This arrangement, accordingly, defines that the part of the measurement passage in the nebulizer housing and the part of the measurement passage in the controller housing align. That is, they have the same central axis, at least at the contacting area of the measurement passage in the nebulizer housing and in the controller housing in a use state of the inhalation therapy device.

Having the pressure-transmitting member close the part of the measurement passage in the controller housing enables that the number of parts in the nebulizer housing, which is to be cleaned after every use, i.e., after every administration interval, can be kept as low as possible, such that the cleaning and/or disinfection can be made as easy as possible. Further, the controller housing having the pressure-transmitting member ensures that, even when the nebulizer housing is detached from the controller housing, the sensor can be protected from the environmental conditions by the pressure-transmitting member.

Vice versa, having the pressure-transmitting member close the part of the measurement passage in the nebulizer housing ensures that any liquids, fluids, or other elements that could negatively affect the operation of the sensor can be kept away from the controller housing, and accordingly the sensor, as far as possible.

Preferably, the position of the nebulizer housing relative to the controller housing in a use state of the inhalation therapy device is fixed via interlocking fasteners. This may include the provision of interacting magnets. The provision of said interlocking fasteners and/or the interacting magnets allows to ensure/fix a gastight connection between the part of the measurement passage in the controller housing and the part of the measurement passage in the nebulizer housing in the use state of the inhalation therapy device.

Such interlocking fasteners can, for example, also be understood as grid-like fastening means, which reliably ensure that the relative position of the nebulizer housing to the controller housing is precisely defined. Doing so, enables to align the central axes of the part of the measurement passage formed in the nebulizer housing to the part of the measurement passage formed in the controller housing. Accordingly, it can be prevented that the gas/air/aerosol flow through the flow path and the interconnected measurement passage gets negatively affected during therapy.

Preferably, the inhalation therapy device comprises two of the pressure-transmitting members. One of the pressure-transmitting members closes the part of the measurement passage in the controller housing and the other one closes the part of the measurement passage in the nebulizer housing.

Having two of such pressure-transmitting members closing each part of the measurement passage in the controller housing and the part of the measurement passage in the nebulizer housing to the environment combines the above-noted advantages: The arrangement at the nebulizer housing provides the hygienic separation, as the controller housing itself must not be cleaned in such detail, and water, droplets, sputum, etc. can be prevented from contacting the controller housing. The pressure-transmitting member in the part of the measurement passage in the controller housing furthermore protects the sensor even when the nebulizer housing is detached from the controller housing.

In this connection, it is to be understood that in a configuration comprising two pressure transmitting members, both can be constituted as a subsequently described non-deflecting body, both can be constituted as a subsequently described flexible pressure-transmitting membrane, or one can be constituted as the non-deflecting body and the other one can be constituted as the flexible pressure-transmitting membrane. In any of said combinations, it may equally be possible that one or both of the two pressure transmitting members is/are replaced by the subsequently described gel. Put differently, the constitution of the two pressure transmitting members can respectively be selected from the non-deflecting body-type, the flexible pressure-transmitting membrane-type, and the gel-type.

Preferably, the aerosol generator is disposed in the flow path between the first opening and the second opening. The connection of the measurement passage and the flow path is disposed upstream of the aerosol generator in the flow path, when seen in the direction of the inhalation flow.

That is, the measurement passage containing the sensor is arranged "behind" the aerosol generator being disposed in the flow path, when seen in the inhalation flow direction. Put differently, it is arranged in a particularly "dry" region in the housing of the inhalation therapy device. Hence, a potential contact of the measurement passage and, accordingly, the pressure-transmitting member with any kind of liquid, sputum, aerosol droplets, rainout, or the like can be kept as low as possible. This improves the measurement quality of the pressure sensor, and, according, the therapy results of the inhalation therapy device.

Yet, the present disclosure is not limited to such a configuration. It may also be possible that the connection of the measurement passage and the flow path is disposed downstream of the aerosol generator in the flow path, when seen in the direction of the inhalation flow.

Preferably, the housing comprises a support surface for supporting the inhalation therapy device on a horizontal surface. That is, for example a part of the housing is shaped in such a manner that the position of the inhalation therapy device can be reliably maintained when being placed on said support surface. The pressure-transmitting member or, when having two pressure-transmitting members, both pressure transmitting members, is/are arranged inclined to said support surface.

Hence, aerosol droplets are prevented from adhering to the pressure-transmitting member(s) such that a deterioration of the measurement quality of the sensor is omitted when the inhalation therapy device is placed, for example, on a table.

On top of that, it may be possible that the pressure-transmitting member(s) are arranged at an elevated position inside the inhalation therapy device to further reduce the risk of an adhesion and an improved drainage of any gems, sputum, and/or aerosol droplets.

According to a further aspect of the present disclosure, the pressure-transmitting member is constituted as a non-deflecting body.

The term non-deflecting is to be understood in that the pressure-transmitting member is shaped and dimensioned in such a manner that the pressure-transmitting member does not oscillate, i.e., deflect, during a regular inhalation (and exhalation) through the flow path of the inhalation therapy device to transmit the pressure generated thereby via the non-deflecting body to the sensor. Instead, the non-deflecting body is impermeable to liquids and permeable to gases, such as air, to transmit the pressure in the flow path to the sensor without the need to be able to deflect for any type of pressure-transmission.

Put differently, the non-deflecting body is a stiff element, such as a disc or plate, which protectively closes the measurement passage towards the sensor but allows the pressure to pass the non-deflecting body itself. That is, it may also be understood as a filter material that enables a pressure transmission yet prevents any liquids from passing the non-deflecting body.

All ranges disclosed in the present application, irrespective of their nature as a pressure range, a diameter range, a thickness range, etc., are to be construed as including their respective upper and lower threshold values.

In this connection, it is preferred that the thickness of the non-deflecting body ranges from 0.1mm to 5mm, preferably from 0.5mm to 3mm, more preferably from 0.5mm to 2.5mm, most preferably from 1mm to 2mm.

Such a thickness of the non-deflecting body allows that the pressure created by the inhalation flow and/or exhalation flow through the flow path can be reliably transferred via the measurement passage through the non-deflecting body to the sensor arranged behind the same in an improved manner: On the one hand, an accidental destruction of the pressure-transmitting member, for example, during the cleaning of the inhalation therapy device, can be omitted, while the measurement quality can be kept as high as possible, because a potential loss in the transmission of the pressure through/via the non-deflecting body can be kept as little as possible.

To achieve the above-described effects and advantages, it is preferred that the non-deflecting body consists of a porous material, which may equally be understood as a foam layer. This porous material may have a pore size in the range of 0.1 to 50 pm, preferably in the range of 2 to 40 pm, more preferably in the range of 3 to 35 µm. Each of said porous material configurations may respectively be provided with a mean pore size of approximately 10µm.

Preferably, the non-deflecting body comprises a thickness of 1.5mm and a gas permeability of 0.01 to 10 m³/min/m² at a pressure differential of 5mbar, preferably 0.1 to 10 m³/min/m² at a pressure differential of 5mbar, more preferably 0.4 to 4 m³/min/m² at a pressure differential of 5mbar.

An example for a material having such properties at a material thickness of 1.5mm is the porous material VYON 1.5M by W. KÖPP GmbH & Co. KG (for easier orientation abbreviated as KÖPP in the subsequent specification), such that it is preferred that the non-deflecting body is constituted by KÖPP's VYON 1.5M material.

It may alternatively also be possible that the non-deflecting body is constituted by KÖPP's VYON 1.5F material. In such a configuration, the non-deflecting body comprises a thickness of 1.5mm and a corresponding gas permeability of 14 m3/min/m2 at a pressure differential of 2.5mbar, and/or 43 m3/min/m2 at a pressure differential of 10mbar, and/or 75 m3/min/m2 at a pressure differential of 20mbar. In such a configuration, the non-deflecting body consists of a porous material having a pore size in the range of 8-12 µm to 95-115µm. More preferably, the pore size is in the above range with a mean pore size in the range of 31-45µm.

The diameter of the non-deflecting body may range from 3mm to 50mm, preferably ranges from 5mm to 20mm, more preferably ranges from 8mm to 12mm, most preferably is 10mm.

These diameters may imply a circular shape of the pressure-transmitting membrane. Yet, the pressure-transmitting membrane of the present disclosure is not limited to a circular shape. Rather, the corresponding surface area resulting from said exemplary diameters is crucial to obtain a reliable and effective pressure transmission through the non-deflecting body while ensuring that the sensor is sufficiently protected. For the sake of completeness, it is noted that the surface area covered by such diameters of the non-deflecting body can easily calculated by π × r² or π × (d/2)².

The non-deflecting body may be made of an appropriate plastic, such as HDPE (high-density-polyethylene) . Preferably the non-deflecting body is made of UHMWPE (ultra-high molecular weight PE), more preferably the non-deflecting body is made of HDPE (high-density-polyethylene), or even more preferably the non-deflecting body is made of PTFE (polytetrafluoroethylene or "teflon") or PE (polyethylene).

It may also be possible that the non-deflecting body is formed of different materials that are equally capable to act as a filter to transmit the pressure but to omit a transmission of any liquids or fluids while being resistant to corrosion. Examples may be synthetic fibers, PE felt, metals, or other porous media.

This allows a simple cleaning without the risk of any bacteria adhering to the pressure-transmitting member surface oriented towards the flow path.

According to an alternative aspect of the present disclosure, the pressure-transmitting member is not a non-deflecting body but a flexible pressure-transmitting membrane instead that is impermeable to liquids and impermeable to gases and that is configured to transmit the pressure in the flow path to the sensor due to a deflection of the pressure-transmitting membrane caused by the pressure in the flow path.

Thus, the flexible pressure-transmitting membrane is to be understood as a membrane that is configured to oscillate/deflect upon the inhalation (and exhalation) through the flow path, which accordingly transmit the pressure to the sensor due to said behaviour, just like a drumhead or like a deflecting body.

Said configuration enables that any liquids and gases can be kept away from the sensor, while the pressure gets consistently transferred through the deflecting membrane, which may, hence, also be understood as a diaphragm.

It is particularly preferred in such a configuration that the diameter of the pressure-transmitting membrane ranges from 3mm to 50mm, preferably from 5mm to 20mm, more preferably from 6mm to 20mm, most preferably from 6mm to 15mm.

This allows to obtain a large active diameter for transmitting the pressure generated by the inhalation/exhalation of the patient trough the flow path and the measurement passage of the inhalation therapy device. Accordingly, solely a little deflection is required, and little damping losses can be achieved.

Preferably, the thickness of the pressure-transmitting membrane ranges from 0.1mm to 2mm, preferably from 0.1mm to 1mm, more preferably from 0.2mm to 1mm, most preferably from 0.3mm to 0.5mm.

This allows to obtain a particularly thin membrane for transmitting the pressure generated by the inhalation/exhalation of the patient trough the flow path and the measurement passage of the inhalation therapy device, which is strong enough to resist the forces acting on the membrane during the cleaning of the inhalation therapy device. That is, said thicknesses ensure a reliably pressure transmission while ensuring that the risk of rupturing the flexible pressure-transmitting membrane can be kept as low as possible.

Preferably, the pressure-transmitting membrane is made of elastomer, optionally thermoplastic elastomer, which is usually abbreviated as TPE, latex, or silicone.

This allows a simple cleaning without the risk of any bacteria adhering to the pressure-transmitting membrane surface oriented towards the flow path. This is particularly the case as having a (T)PE or silicone membrane results in a smooth surface that is easy and reliably cleanable. Further, the costs for the pressure-transmitting membrane can be kept as low as possible.

Suitably, a volume between the pressure-transmitting membrane and the sensor is larger than 1mm³, preferably larger than 5mm³, and is less than 1600mm³, preferably less than 1000mm³, more preferably less than 800 mm³.

That is, the pressure-transmitting membrane and the sensor are arranged in a distanced manner, such that a volume between said components is given. Said distance is to be understood as the length of a normal onto the flexible pressure-transmitting membrane surface to the sensor surface arranged in/at the end of the measurement passage, i.e., behind the pressure-transmitting membrane when seen from the flow path of the inhalation therapy device.

Having such a volume between the pressure-transmitting membrane and the sensor leads to a decoupling of the measurement surface of the sensor and the pressure-transmitting membrane itself, such that the pressure-transmitting membrane can swing freely upon being actuated by the pressure. In this connection, having a small volume between the pressure-transmitting membrane and the sensor allows keeping potential pressure transfer losses as low as possible. This is the case, as the amount of gas, especially air, as a compressible medium, filling said volume is kept as low as possible. The lower said locked volume, the lower the volume change upon a change of the to be transferred pressure and the lower the deflection of the pressure transmitting membrane, and accordingly, the lower a deterioration of the measurement quality.

Preferably, and to ensure that the loss in the transmission of the pressure in the flow path and the interconnected measurement passage via the flexible pressure-transmitting membrane to the pressure sensor, the hardness of the pressure-transmitting membrane ranges from 10 to 100 shore A.

Having such a low stiffness ensures that potential losses in the transmission of the pressure from the one side of the flexible pressure-transmitting membrane being positioned towards the flow path to the other side of the flexible pressure-transmitting membrane being arranged towards the sensor in the measurement passage can be kept as low as possible. Thus, a reliable control of the inhalation therapy device and, consequently, the therapy itself can be obtained.

According to a further alternative aspect of the present disclosure, the pressure-transmitting member is neither a non-deflecting body nor a flexible pressure-transmitting membrane but is constituted as a liquid- and gas impermeable gel that is configured to transmit the pressure in the flow path to the sensor.

Such a configuration allows to protect the sensor from any environmental pollution while transferring the pressure to be detected by the sensor in a consistent and reliable manner.

According to an even further alternative aspect of the present disclosure, the pressure transmitting member may, in case of having one pressure transmitting member, either be a non-deflecting body or the flexible pressure-transmitting membrane, and the part of the measurement passage between the pressure-transmitting member and the sensor is filled, especially substantially entirely filled, by the liquid- and gas impermeable gel. For example the gel comprises a very high viscosity which is liquid- and gas impermeable.

In a case of having two pressure transmitting members, wherein both are constituted by the non-deflecting body, both are constituted by the flexible pressure-transmitting membrane, or one is constituted as the non-deflecting body and the other one is constituted as the flexible pressure-transmitting membrane, both parts of the measurement passage may be filled, by the liquid- and gas impermeable gel.

This reduces potential losses in transferring the pressure to the pressure sensor, as, for example, air being a compressible medium is replaced by the gel. More preferably, the gel may be biocompatible.

### Brief Description of the Drawings

Hereinafter, non-limiting examples of the disclosure are explained with reference to the drawings, in which:
Figure 1 shows an embodiment of the inhalation therapy device according to the present disclosure in an perspective view.
Figure 2 shows a schematic cross-sectional view along the flow path of the inhalation therapy device of Figure 1.
Figure 3 shows a frontal cross-sectional view on the aerosol generator of the inhalation therapy device, wherein the cross-section is taken perpendicular to the first flow direction.
Figure 4 shows an exploded view of the nebulizer housing, which is connectable to the additionally illustrated controller housing, which together form the inhalation therapy device of Figure 1.
Figure 5 shows a schematic enlarged partial cross-sectional view through a part of the nebulizer housing and a part of the controller housing for illustrating the respective configuration and arrangement of the pressure-transmitting members in the nebulizer housing and the controller housing.

### Detailed Description of Preferred Embodiments

A currently preferred embodiment of the present disclosure regarding an inhalation therapy device 1 will now be described with reference to the accompanying drawings. Such inhalation therapy devices 1 are oftentimes also called "aerosol delivery devices" or "nebulizers", for example, for a therapeutic use via a user's respiratory system.

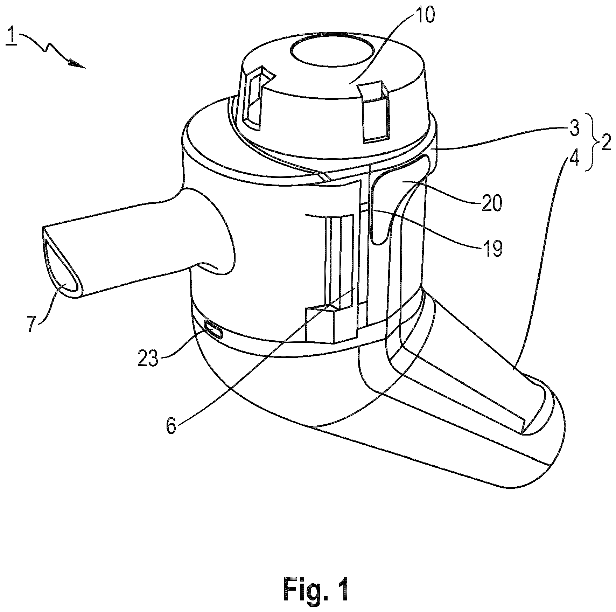

Fig. 1 shows an exemplary isometric view of the inhalation therapy device 1 according to a currently preferred embodiment of the present disclosure.

The overall shape of the inhalation therapy device 1 is defined by its housing 2, which is split into two main parts. The housing 2 comprises a nebulizer housing 3, which may equally be construed as an aerosolization portion, and a controller housing 4, which may also be called controller portion or holding portion.

Yet, the present disclosure is not limited to such a split configuration: It may also be possible to form the nebulizer housing 3 and the controller housing 4, which constitute the housing 2 of the present embodiment, as an integral element. Such a configuration will, however, not be discussed in more detail here.

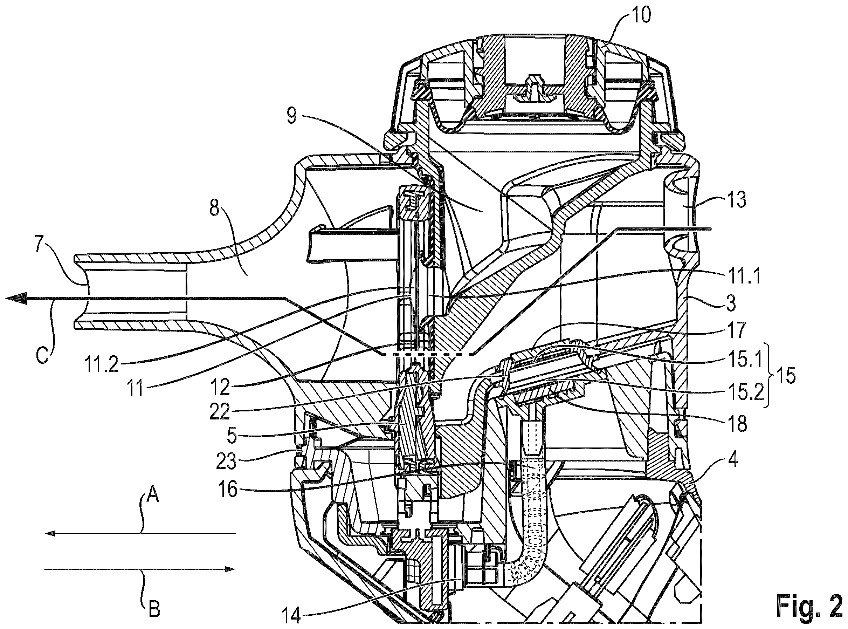

The nebulizer housing 3 accommodates an aerosol generator 5 configured to generate an aerosol from a liquid or fluid, i.e., a medicament, which will be described in more detail with reference to Figs. 2, 3, and 5 below.

The controller housing 4 generally aims to enable that the inhalation therapy device 1 can be held by a user during (aerosol) therapy and contains the most relevant, preferably all, electronic features and sensory elements necessary to operate the inhalation therapy device 1 and to monitor the therapy process.

Accordingly, the controller housing 4 can be construed as a long-lasting element, which has lower requirements regarding cleaning and/or disinfection compared to the nebulizer housing 3, which can be configured as a throwaway product or a product, which is to be used for a certain amount of time, such as a certain number of therapies, weeks, months, or years. For example the nebulizer housing 3 and the aerosol generator 5 can be replaced every one month, every three months or every year (year pack).

Hitherto, the position of the nebulizer housing 3 relative to the controller housing 4 in a use state of the inhalation therapy device 1 is at least temporarily fixed via interlocking fasteners, and it is thereby also possible to reversibly attach and detach the nebulizer housing 3 from the controller housing 4 for the ease of cleanability of both parts of the housing 2 and replacement of the nebulizer housing 3 via said interlocking fasteners. It is apparent from Figures 2, 4, and 5 that said interlocking fasteners may, for example, be realized by way of a spring hook 23. Said illustrated spring hook 23, which is exemplarily arranged at a front section of the controller housing 4, interacts with a recess formed in the nebulizer housing 3 to secure the relative position of the nebulizer housing 3 to the controller housing 4 during use of the inhalation therapy device 1. Hence, having such a spring hook 23 enables that opening mechanisms are no longer needed.

To facilitate obtaining the correct relative position of the controller housing 4 and the nebulizer housing 3, the preferred embodiment of the present disclosure additionally comprises non-illustrated interacting magnets. These are respectively arranged in the controller housing 4 and the nebulizer housing 3 and thereby support in bringing the controller housing 4 and the nebulizer housing 3 in a position, where the interlocking fasteners (temporarily) fix the relative position of these two housing parts during use of the inhalation therapy device 1 and secure the below-described gas tight connection of the two parts 15.1 and 15.2 of the measurement passage in the nebulizer housing 3 and the controller housing 4.

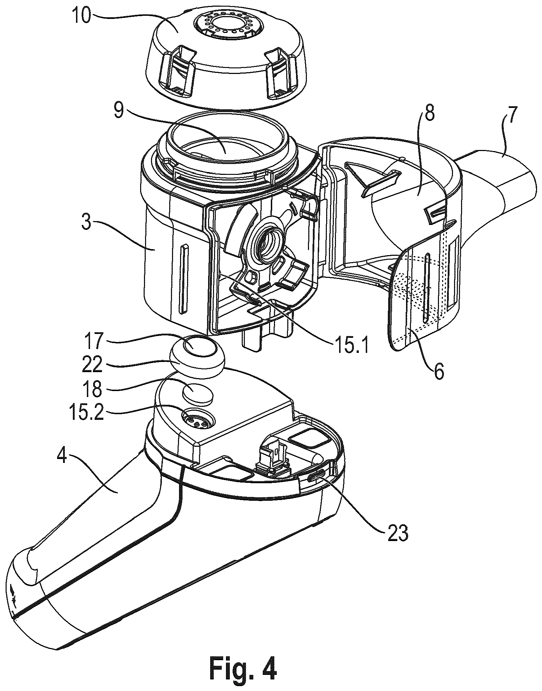

To enable a decent cleanability and/or disinfection of the nebulizer housing 3 and an easy access of the aerosol generator 5 arranged inside the nebulizer housing 3, it is apparent from Figures 1 and 4 that a latch 6 is provided at the nebulizer housing 3. The latch 6 enables access to the interior of the nebulizer housing 3, especially to the aerosol generator 5 arranged therein. Hitherto, Fig. 1 shows the inhalation therapy device 1 when the latch 6 holds the nebulizer housing 3 in a closed state and Fig. 4 shows the same in an open state, such that the (in Fig. 4 non-illustrated) aerosol generator 5 can be accessed. Yet, providing the latch 6 is not compulsory. It is also possible to provide a nebulizer housing 3 that cannot be opened by a user, operator, hospital staff, or the patient itself. This is, for example, achievable by forming a nebulizer housing 3, in which the aerosol generator 5 is arranged in a fixed position and cannot be accessed/replaced.

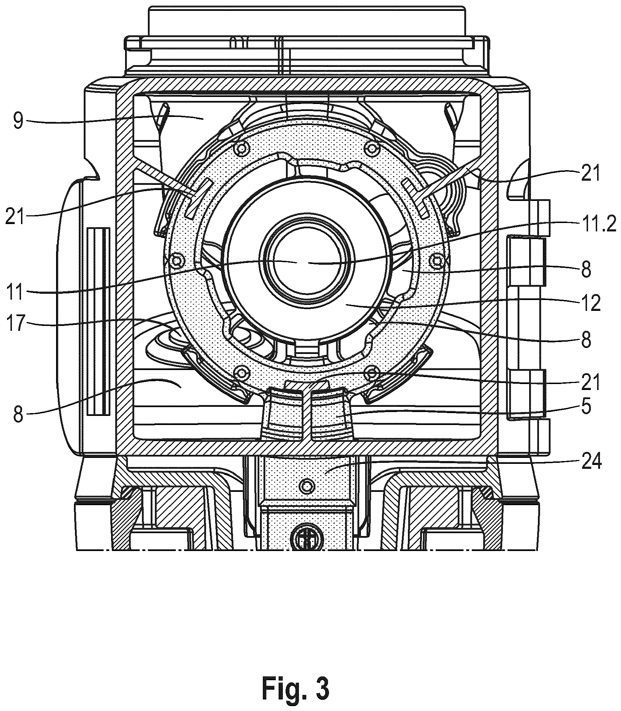

In the technical field given, such aerosol generators 5, as illustrated, among others, in Figure 3, are oftentimes known as membrane units or head units. Similarly, the nebulizer housing 3 is known as a "nebset", which reflects an abbreviation of nebulization set. Alternatively, the "nebset" is also known as a "mouth piece assembly" (MPA), which includes the membrane unit (also known as head unit) as well as the mouth piece for the user.

The preferred embodiment is an inhalation therapy device 1 through which a user inhales and exhales during the therapy. That is, the inhalation therapy device 1 is brought into contact with the user's mouth, for example via a mouth piece, or mouth and nose, for example, via a (face) mask, such that the regular inhalation and exhalation of the user is performed through the inhalation therapy device 1. That is, the inhalation therapy device 1, specifically its first opening 7 (which will be described in more detail below) shall not be removed from the user's mouth (and nose) during the aerosol therapy.

How the aerosol is generated inside the inhalation therapy device 1 and how the actual therapy is to be performed will now be described in more detail.

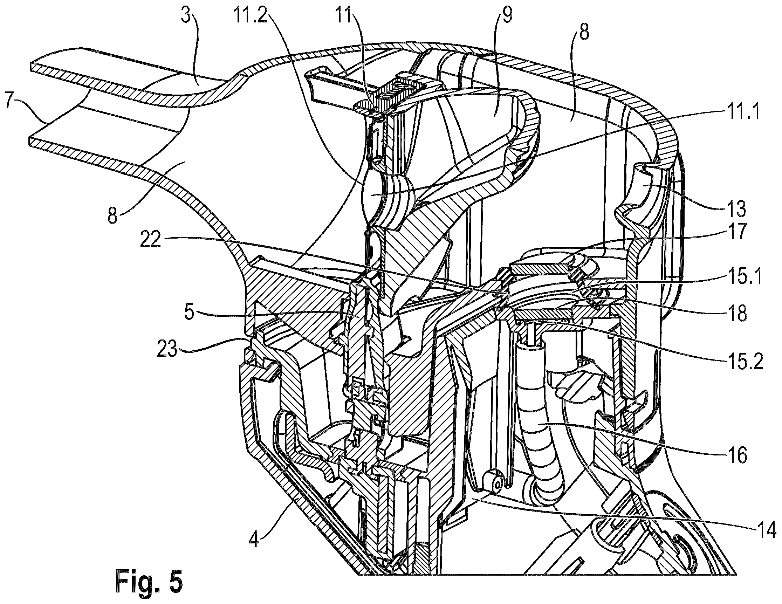

Fig. 2 illustrates the orientation of the inhalation therapy device 1 when it is held by a patient in an ideal position during therapy, i.e., in the sitting position of the patient and an upright position of the patient's head. For orientation purposes, solely the uppermost section of the controller housing 4, when the inhalation therapy device 1 is held in said ideal position, is illustrated. At the same time, Fig. 2 shows a cross-sectional view through the inhalation therapy device 1 along a flow path 8 and Figure 5 shows a schematic enlarged partial cross-sectional view through a part of the nebulizer housing and a part of the controller housing for illustrating the respective configuration and arrangement of the pressure-transmitting members in the nebulizer housing and the controller housing. It is, in this connection, illustrated that the nebulizer housing 3 contains the flow path 8, which passes through said nebulizer housing 3 and that the aerosol generator 5 is disposed in said flow path 8.

In the illustrated position of Figures 2 and 5, a reservoir 9 for holding a liquid or fluid (i.e., the medicament) is provided at an upper section of the nebulizer housing 3. Further, a lid 10 is provided on the upper section of the reservoir 9 for ensuring that the fluid or liquid in the reservoir 9 is not spilled during therapy.

To produce the aerosol for therapy, the aerosol generator 5 arranged in the flow path 8 through the nebulizer housing 3 comprises a membrane unit, which comprises an aerosol-generating membrane 11 and an actuator 12, which are connectable via a plug 24. This is illustrated in more detail in Figure 3. Vice versa, the membrane unit is not illustrated in Figure 4 for easier orientation. As is apparent, for example, from said Figure 3, the actuator 12 may be constituted as a piezo-electric ring or oscillating generator. In Figure 3, the actuator 12 is a piezo crystal, which surrounds the aerosol-generating membrane 11 in a circular manner and is glued to a substrate.

In the cross-sectional view of Fig. 2, the aerosol-generating membrane 11 is arranged in an upright position, i.e., in a vertical position, in the nebulizer housing 3, such that the aerosol-generating membrane 11 can encounter the fluid or liquid being held in the reservoir 9. In other words, the reservoir 9 and the aerosol-generating membrane 11 are disposed adjacent to each other, such that the fluid or liquid can be supplied to a first side 11.1 of the aerosol-generating membrane 11, when a fluid or liquid is held in the reservoir 9. Doing so enables that the fluid or liquid is automatically, i.e., gravitationally, transported to the aerosol-generating membrane 11 of the aerosol generator 5 for producing aerosol for the user's therapy. This is achieved by arranging the aerosol-generating membrane 11 at a lowermost position of the reservoir 9 when the inhalation therapy device 1 is held by a user during therapy (see Fig. 2).

As is apparent from Figure 3, the aerosol-generating membrane 11 comprises a plurality of (non-illustrated) apertures and the actuator 12 is coupled to the aerosol-generating membrane 11, such that it can vibrate the same. By doing so, the liquid passes through the apertures, and aerosol is generated at a second side 11.2 of the aerosol-generating membrane 11 opposite of the first side 11.1 thereof. That is, an aerosol bolus, which may also be understood as an "aerosol plume", is generated in the chamber on the in Fig. 2 left-hand side of the aerosol generator 5 inside the nebulizer housing 3.

To entrain and deliver the generated aerosol at the second side 11.2 of the aerosol-generating membrane 11 inside the nebulizer housing 3 of the inhalation therapy device 1 to the user's respiratory tract, the inhalation therapy device 1 comprises the flow path 8.

The flow path 8 comprises a first opening 7 to the outside of the housing 2, particularly the nebulizer housing 3 at one end thereof and a second opening 13 to the outside of the housing 2, particularly the nebulizer housing 3 at another end of said nebulizer housing 3.

In the illustrated embodiment of Fig. 2, the first opening 7 of the flow path 8 and the second opening 13 are arranged at opposing sides of the nebulizer housing 3. Yet, said arrangement is not binding and it may also be possible that, for example, the position of the second opening 13 is arranged offset from a longitudinal axis through the first opening 7 of the flow path 8.

As mentioned earlier, it is the first opening 7 that is to be brought into contact, may it be directly or indirectly, for example, via a mouthpiece or a mask, with the user's respiratory tract via his or her mouth, and optionally his or her nose, to enable inhalation and exhalation through the flow path 8 of the inhalation therapy device 1 during the aerosol therapy.

Vice versa, the second opening 13 allows air to enter the flow path 8 and to be expelled therefrom. Hence, the flow path 8 and its first opening 7 and second opening 13 to the outside of the nebulizer housing 3 enable that a flow is generatable in the first flow direction A from the second opening 13 to the first opening 7 in the flow path 8 upon inhalation of the user at the first opening 7. This flow may be understood as an inhalation flow, wherein the entire flow volume along the first flow direction A to the first opening 7 passes through the second opening 13 into the flow path 8 past a measurement passage 15 (which will be described in more detail below) and the aerosol generator 5, and merges with the aerosol at the second side 11.2 of the aerosol-generating membrane 11 before it gets expelled from the inhalation therapy device 1 into the user's body at the first opening 7 of the flow path 8.

In the embodiment of Fig. 2, also the entire flow volume along the second flow direction B, i.e., the direction of the exhalation flow from the first opening 7 to the second opening 13, passes through the second opening 13 upon exhalation of the user at the first opening into the flow path 8.

Fig. 2 illustrates the first flow direction A from the second opening 13 to the first opening 7, i.e., the direction of the inhalation flow, and the second flow direction B from the first opening 7 to the second opening 13 in the flow path 8, i.e., the direction of the exhalation flow.

Regarding the shape of the flow path 8, it is apparent from Fig. 2 that the flow path 8 is shaped to prohibit a direct (un-redirected) streaming of air from the second opening 13 to the first opening 7 of the flow path 8 and vice versa. Such an exemplary flow through the flow path 8 in the nebulizer housing 3 is illustrated by line C in Fig. 2. It is apparent therefrom that a centre point of the cross-section of the first opening 7, a centre point of the cross-section of the aerosol generator 5, and a centre point of the cross-section of the second opening 13 do not align with each other in one single axis, thereby provoking a redirection of the air through the inhalation therapy device 1, and thereby omitting a pipe-shaped flow path structure.

As is apparent from the frontal cross-sectional view on the aerosol generator 5 of the inhalation therapy device 1 of Figure 3, the aerosol generator 5 is disposed in the flow path 8, and thereby in said flow generatable by the user's breathing, especially his or her inhalation and exhalation, in such a manner that it can be surrounded by the flow through the flow path 8. As illustrated in Figure 3, the aerosol-generating membrane 11 is arranged and held in an axially and circumferentially centred position of the flow path 8 via three webs 21, such that a sheathing enveloping flow can pass the entire active area of the aerosol generator 5 inside the flow path 8 during inhalation and exhalation of the user by breathing through the inhalation therapy device 1 during therapy.

The line C in Figure 2 extending from the second opening 13 to the first opening 7 represents an exemplary flow of the air along the first flow direction A (i.e., an inhalation (air) flow). It should, however, be understood that this exemplary flow path 8 is not limited to the flow passing underneath the aerosol generator 5 passed a lowermost position of the flow path 8 as well as through the aerosol generator 5 towards the first opening 7. This is merely one example of a flow through the cross-sectional illustration of the flow path 8. In reality, the flow is a sheathing enveloping flow (see Figure 3), which passes the aerosol generator 5 and the illustrated reservoir 9, for example, also on the sides of the aerosol generator 5, as it is arranged in such a manner that an envelope flow is generated about the circumference of aerosol-generating membrane 11 of the aerosol generator 5.

It is the air flow that is redirected to be guided around the aerosol-generating membrane 11 of the aerosol generator 5 for entraining and delivering the generated aerosol upon inhalation.

To trigger the actuator 12 of the aerosol generator 5 and to evaluate the quality of the inhalation therapy, it is required to observe the way the inhalation and the exhalation through the inhalation therapy device 1 is performed. Hitherto, the preferred embodiment provides an easy to manufacture and disinfect solution that reliably monitors/measures the pressure and negative pressure inside the flow path 8 upon inhalation and exhalation as a flow parameter inside the flow path 8.

To do so, the inhalation therapy device 1 comprises a sensor 14, which is arranged in the controller housing, and which is configured to detect a pressure value inside the flow path 8.

It is apparent from the illustration of the inhalation therapy device 1 in Figures 2 and 5 that the controller housing 4 accommodates said sensor 14 besides a non-illustrated controller, which is configured to control the aerosol generator 5, i.e., which is configured to trigger the actuator 12 for generating the aerosol for therapy. Accordingly, the sensor 14 arranged in the controller housing 4 is positioned outside the flow path 8 extending through the nebulizer housing 3.

To provide the observation of the pressure values in the flow path 8 via the sensor 14, the inhalation therapy device 1 comprises a measurement passage 15, which connects the flow path 8 and the sensor 14. Given the fact that the flow path 8 extends through the nebulizer housing 3 and the sensor 14 is securely housed in the controller housing 4, the measurement passage 15 comprises a part 15.1 being formed in the nebulizer housing 3 and a part 15.2 being formed in the controller housing 4. In the preferred embodiment, the entire measurement passage 15 comprises a circular shape and the central axes of the part 15.1 being formed in the nebulizer housing 3 and the part 15.2 being formed in the controller housing 4 align with each other, such that the part 15.1 being formed in the nebulizer housing 3 and the part 15.2 being formed in the controller housing 4 form one single gas tight cylindrical channel, a hose, a pipe, or the like, which interconnects the flow path 8 and the sensor 14 without disturbing the flow mitigation in the measurement passage 15 by turbulences or the like. Accordingly, the pressures created by the inhalation and exhalation of the patient can mitigate through the flow path 8 and through the measurement passage 15 in order to be detected by the sensor 14. In the exemplary embodiment given, the part 15.1 being formed in the nebulizer housing 3 and the part 15.2 being formed in the controller housing 4 achieve said gas tightness by way of a sealing lip 22. Nonetheless, the gas tightness between the part 15.1 being formed in the nebulizer housing 3 and the part 15.2 being formed in the controller housing 4 in the use said of the inhalation therapy device may equally be achievable by the provision of O-Rings or by way of a form fit of the part 15.1 being formed in the nebulizer housing 3 and the part 15.2 being formed in the controller housing 4.

As is apparent, for example, from Figure 5, the sealing lip 22 of the exemplary embodiment accommodates the pressure-transmitting member 17 and is positionally fixed in the nebulizer housing 3 by way of having two undercuts along the axial direction of the sealing lip 22. Thus, the sealing lip 22 combines the function of fixing the position of the pressure-transmitting member 17 in the part 15.1 of the measurement passage 15 being formed in the nebulizer housing and the function of gas-tightly sealing said part 15.1 with the part 15.2 arranged inside the controller housing 4.

The connection of the measurement passage 15, particularly the part 15.1 of the measurement passage 15 in the nebulizer housing 3 and the flow path 8 is disposed upstream of the aerosol generator 5 in the flow path 8, when seen in the direction of the inhalation flow A. To illustrate the same, the aerosol generator 5 is hidden in the open state of the nebulizer housing 3 illustrated in Fig. 4. Figures 2 and 5 illustrated said configuration as well.

As illustrated in Figs. 2 and 5, the provision of the measurement passage 15 allows a distant arrangement of the sensor 14 from the flow path 8. The pressure sensor 14 of the exemplary embodiment is a differential pressure sensor that determines a static pressure in the flow path 8 and compares the same to the environmental pressure, i.e., a reference pressure, which may also be understood as an ambient pressure. To detect said environmental pressure, the controller housing 4 may additionally comprise a hose 16 extending through the controller housing 4 that enables a detection of said environmental pressure and a comparison of the same with the pressure in the flow path 8 via the differential pressure sensor 14. In such a scenario, the hose 16 is a part of the measurement passage 15, particularly a part 15.2 of the measurement passage in the controller housing 4.

In the exemplary embodiment, not only the measurement passage 15, but also the second opening 13 of the flow path 8 is arranged upstream of the aerosol generator 5, when seen in the first flow direction A. It is, in this connection, apparent from Figs. 2, 4 and 5 that the second opening 13 is arranged at an upper half of the nebulizer housing 3.

It is furthermore apparent from the cross-sectional views of Figs. 2 and 5 that the measurement passage 15 is disposed substantially in the middle between the aerosol generator 5 and the second opening 13 in the flow path 8, when seen along the first flow direction A. However, this arrangement is not particularly binding, and it may also be possible to arrange the interconnection of the measurement passage 15 and the flow path 8 closer to the second opening 13 than to the aerosol generator 5 in the flow path 8 or even to arrange the measurement passage 15 closer to the aerosol generator 5 than to the second opening 13 in the flow path 8.

To protect the sensor 14, which is connected to the flow path 8 via the measurement passage 15, from liquids, aerosol (droplets), sputum, or the like in the best possible way and to allow a secure and reliable cleaning of the inhalation therapy device 1 even when the nebulizer housing 3 and the controller housing 4 are detached from each other, the inhalation therapy device 1 according to the illustrated, preferred embodiment of the present disclosure comprises two pressure-transmitting members 17, 18. One pressure-transmitting membrane 17 closes/seals the part 15.1 of the measurement passage 15 in the nebulizer housing 3, while the other pressure-transmitting membrane 18 closes/seals the part 15.2 of the measurement passage 15 in the controller housing 4.

In this connection, the pressure-transmitting members 17, 18 are configured to transmit the pressure in the flow path 8 to the sensor 14 while being impermeable to liquids. In this connection, both pressure-transmitting members 17, 18 comprise a cylindrical basic structure with hydrophobic properties on their respective surfaces oriented towards the interconnection region of the controller housing 4 and the nebulizer housing 3 to prohibit the adherence of any liquids, fluids, sputum, etc. on the pressure-transmitting members 17, 18, which could potentially affect or even adulterate the pressure-transmission through the measurement passage 15 to the sensor 14 via the pressure-transmitting members 17, 18 (see Figures 2 and 5).

Each of the pressure-transmitting members 17, 18 of the illustrated embodiment is constituted as a stiff, non-deflecting body, which closes the respective part 15.1, 15.2 of the measurement passage 15 and allows the pressure transmission due to its liquid impermeability but gas permeability, particularly air permeability.

Hitherto, the respective non-deflecting body of the present embodiment consists of KÖPP VYON 1.5M material with a thickness of 1.5mm. That is, the non-deflecting body consists of a porous (filter) material mode of PE having a pore size in the range of 5 to 35 µm with a mean pore size of 10 µm at a thickness of 1.5mm. At said thickness, the non-deflecting body is provided with a gas permeability of 3 m3/min/m2 at a pressure differential of 5mbar, and/or 6 m3/min/m2 at a pressure differential of 10mbar, and/or 13 m3/min/m2 at a pressure differential of 20mbar.

Independent thereof, the non-deflecting body comprises a diameter of 10mm, but is not limited to said exemplary diameter. Due to the characteristics of the pressure-transmitting members 17, 18 being respectively constituted as a non-deflecting body acting as a filter for liquids, the diameter, and thereby the active area for transmitting the pressure through the respective pressure transmitting member 17, 18 can vary dependent on the device size, the environmental conditions, and/or the intended use.

It is apparent from Figs. 2 and 5 that the sensor 14 in the controller housing 4 is not in direct contact with the pressure-transmitting membrane 18, which closes the part 15.2 of the measurement passage 15 in the controller housing 4 between the sensor 14 and said pressure-transmitting membrane 18. To the contrary, the pressure-transmitting membrane 18 and the sensor 14 are distant to each other. In the exemplary embodiment illustrated in the drawings said volume is 775mm³.

However, the present disclosure is not limited to a configuration of the pressure-transmitting members 17, 18 being constituted by a non-deflecting body.

In contrast, one or both of the non-deflecting bodies of the pressure-transmitting members 17, 18 can be replaced by a liquid- and gas impermeable gel that is configured to transmit the pressure in the flow path 8 to the sensor 14.

As an even further alternative, one or both of the non-deflecting bodies of the pressure-transmitting members 17, 18 can be replaced by a flexible pressure-transmitting membrane that is impermeable to liquids and gases and that is, at the same time, configured to transmit the pressure in the flow path 8 to the sensor 14 due to a deflection, i.e., an oscillation, of the respective pressure-transmitting membrane caused by the pressure in the flow path 8.

In this scenario, the active area of the pressure transmitting membrane, that is, for example, formed of TPE, should be formed as large as possible, such that the diameter could, for example, be chosen to be 10mm.

Other than the non-deflecting body mentioned above, the flexible pressure-transmitting membrane should be formed as thin as possible to obtain a reliable deflection of minimal losses upon the inhalation and exhalation through the inhalation therapy device 1, while still being strong enough to avoid any ruptures upon cleaning. Thus, the thickness of the of the pressure-transmitting membrane form the pressure-transmitting member 17 and/or the pressure-transmitting member 18 in such an exemplary embodiment may, for example be 0.4mm, 0.5mm, or 0.6mm.

Also the hardness of the pressure-transmitting membranes may range from 10 to 100 shore A in the given context and may exemplary be selected to be 80 shore A for both pressure-transmitting membranes.

Yet, the present disclosure is not limited to such a configuration of the thickness, the diameter and the material of the flexible pressure-transmitting membrane. To keep the above-discussed losses due to the pressure transmission through the pressure-transmitting membranes 17, 18 as low as possible, it may equally be possible to adapt the thickness, hardness, diameter and/or material configuration of one or both pressure-transmitting membranes 17, 18. That includes, that the pressure-transmitting membrane 17 in the nebulizer housing 3 comprises a different constitution than the pressure transmitting membrane 18 in the controller housing 3. The same holds, of course, equally true for the constitution of the non-deflecting bodies discussed above.

The second opening 13 may be shaped in such a manner that it can be considered as a flow restrictor. To achieve the flow resistance, a geometrical form is to be established that provokes a higher flow resistance in the inhalation flow direction than in the exhalation flow direction. Typical relationships of the inhalation flow resistance to the exhalation flow resistance established by the second opening's shape are 125%, 130%, 150%, 175%, 190% or even 300%, 400%, or 500%. In a most preferred embodiment, the relationship of the inhalation flow resistance to the exhalation flow resistance established by the second opening's shape is 190%. This enables to advantageously prolong the inhalation time, while the exhalation time can be shortened.

The actual shape of the second opening 13, which is responsible to consider it as the above-mentioned flow restrictor, will now be described in more detail with reference to Fig. 2.

To do so, the flow path's second opening 13 is shaped as a fixed geometry passive valve. The second opening 13 may be formed as an integral part of the nebulizer housing 3 or as a separate structural element being connected to the nebulizer housing 3 and forming the outlet of the flow path 8 through said nebulizer housing 3 for delivering the generated aerosol to the user during therapy.

It can be taken from Fig. 2 that the cross-sectional shape of the second opening 13 continuously decreases along the second flow direction B. This continuously decreasing cross-sectional shape of the second opening 13 along the second flow direction B enables that the flow resistance in the second flow direction B can be made lower than in the first flow direction A without the need of any active elements such as actuated valves or the like.

In another embodiment, it may also be possible that another, third opening 19 to the outside of the nebulizer housing 3 is provided in the flow path 8 between said first opening 7 and the second opening 13.

This is, for example, illustrated in Fig 1. The third opening 19 is arranged upstream of the aerosol generator 5 when seen in the first flow direction A. Further, the third opening 19 is provided with a check valve 20, which is configured to restrict a flow through the third opening 19 in the first flow direction A. That is, the check valve 20 prohibits any flow through the third opening 19 of the flow path 8 upon inhalation. However, the check valve 20 is also configured to enable a flow through the third opening 19 in the second flow direction B from the first opening 7 to the second opening 13 for enabling at least a partial, preferably substantially a full expel of air through the third opening 19.

This configuration allows to achieve the above-desired inhalation flow resistance and, at the same time, allows to reduce the exhalation flow resistance even further, as the check valve 20 facilitates the expel of air via the check valve 20 and the third opening 19 of the flow path 8.

Irrespective of the presence of a third opening 19 or not, the inhalation therapy device 1 may, in a further, not illustrated embodiment, comprise an air filter device, which is reversibly attachable and detachable to the nebulizer housing 3. Said air filter device is configured to filter harmful substances from the exhalation flow. The exhalation flow may contain substances and/or aerosols, which may contain materials or germs that may be harmful for the environment. Provided that the third opening 19 is present, the air filter device is shaped to cover also said third opening 19 and its check valve 20, such that the flow in the second flow direction B primarily/also passes through the air filter device to the outside of the nebulizer housing 3 upon exhalation. Vice versa, if the third opening 19 is not present and the flow path 8 extends between the first opening 7 and the second opening 13 only, the air filter device is shaped to cover the second opening 13, which is, besides the first opening 7, the only opening of the flow path 8 to the outside of the nebulizer housing 3, such that the entire flow volume along the first flow direction A passes through the air filter device upon inhalation, and, upon exhalation, the entire flow volume along the second flow direction B passes through the air filter device.

### Reference Signs List

- 1: inhalation therapy device
- 2: housing
- 3: nebulizer housing
- 4: controller housing
- 5: aerosol generator
- 6: latch
- 7: first opening of the flow path
- 8: flow path
- 9: reservoir
- 10: lid
- 11: aerosol-generating membrane
- 11.1: first side of the aerosol-generating membrane
- 11.2: second side of the aerosol-generating membrane
- 12: actuator
- 13: second opening of the flow path
- 14: sensor (pressure sensor)
- 15: measurement passage
- 15.1: part of the measurement passage in the nebulizer housing
- 15.2: part of the measurement passage in the controller housing
- 16: hose
- 17: pressure-transmitting member in the nebulizer housing
- 18: pressure-transmitting member in the controller housing
- 19: third opening
- 20: check valve
- 21: web
- 22: sealing lip
- 23: spring hook
- 24: plug
- A: first flow direction (inhalation flow direction)
- B: second flow direction (exhalation flow direction)
- C: exemplary flow through the flow path

## Claims

1. Inhalation therapy device (1), comprising:
a housing (2),
an aerosol generator (5) configured to generate an aerosol from a liquid,
a flow path (8) being defined in the housing (2) and having a first opening (7) to the outside of the housing (2) at one end and a second opening (13) to the outside of the housing (2) at another end, so that at least an inhalation flow (A) from the second opening (13) to the first opening (7) is generatable in the flow path (8) upon inhalation of a patient at the first opening (7) for entraining and delivering the generated aerosol,
a measurement passage (15) connecting the flow path (8) and a sensor (14), wherein the sensor (14) is positioned outside the flow path (8) and is configured to detect a pressure in the flow path (8) via the measurement passage (15), and
a pressure-transmitting member for transmitting the pressure in the flow path (8) to the sensor (14),
wherein the pressure-transmitting member (17, 18) closes the measurement passage (15) and is impermeable to liquids.

2. Inhalation therapy device (1) according to claim 1, wherein at least a surface of the pressure-transmitting member (17, 18) being oriented away from the sensor (14) comprises hydrophobic properties.

3. Inhalation therapy device (1) according to any of the preceding claims, wherein the housing (2) comprises:
a nebulizer housing (3) defining the flow path (8) and accommodating the aerosol generator (5), and
a controller housing (4) accommodating a controller configured to control the aerosol generator (5), and the sensor (14),
wherein the nebulizer housing (3) is detachable from the controller housing (4),
wherein a part (15.1) of the measurement passage (15) is formed in the nebulizer housing (3) and a part (15.2) of the measurement passage (15) is formed in the controller housing (4), and
wherein the pressure-transmitting member (17, 18) closes the part (15.2) of the measurement passage (15) in the controller housing (4) or the part (15.1) of the measurement passage (15) in the nebulizer housing (3).

4. Inhalation therapy device (1) according to claim 3, wherein the position of the nebulizer housing (3) relative to the controller housing (4) in a use state of the inhalation therapy device (1) is fixed via interlocking fasteners and/or interacting magnets to ensure a gastight connection between the part (15.2) of the measurement passage (15) in the controller housing (4) and the part (15.1) of the measurement passage (15) in the nebulizer housing (3) in the use state of the inhalation therapy device (1).

5. Inhalation therapy device (1) according to claim 3 or 4, wherein the inhalation therapy device (1) comprises two of the pressure-transmitting members (17, 18), wherein one closes the part (15.2) of the measurement passage (15) in the controller housing (4) and the other one closes the part (15.1) of the measurement passage (15) in the nebulizer housing (3).

6. Inhalation therapy device (1) according to any of the preceding claims, wherein the aerosol generator (5) is disposed in the flow path (8) between the first opening (7) and the second opening (13) and the connection of the measurement passage (15) and the flow path (8) is disposed upstream of the aerosol generator (5) in the flow path (8), when seen in the direction of the inhalation flow (A).

7. Inhalation therapy device (1) according to any of the preceding claims, wherein the housing (2) comprises a support surface for supporting the inhalation therapy device (1) on a horizontal surface, and
wherein the pressure-transmitting member (17, 18) is arranged inclined to the support surface.

8. Inhalation therapy device (1) according to any of the preceding claims, wherein the pressure-transmitting member (17, 18) is a non-deflecting body being impermeable to liquids and permeable to gases for transmitting the pressure in the flow path (8) to the sensor (14).

9. Inhalation therapy device (1) according to claim 8, wherein the thickness of the non-deflecting body ranges from 0.1mm to 5mm, preferably from 0.5mm to 3mm, more preferably from 0.5mm to 2.5mm, most preferably from 1mm to 2mm.

10. Inhalation therapy device (1) according to claim 8 or 9, wherein the non-deflecting body consists of a porous material having a pore size in the range of 0.1 to 50 pm, preferably in the range of 2 to 40 pm, more preferably in the range of 3 to 35 pm, most preferably of approximately 10 µm.

11. Inhalation therapy device (1) according to claim 9 or 10, wherein the non-deflecting body comprises a thickness of 1.5mm and a gas permeability of 0.01 to 10 m³/min/m² at a pressure differential of 5mbar, preferably 0.1 to 10 m³/min/m² at a pressure differential of 5mbar, more preferably 0.4 to 4 m³/min/m² at a pressure differential of 5mbar.

12. Inhalation therapy device (1) according to any of the preceding claims 8 to 11, wherein the diameter of the non-deflecting body ranges from 3mm to 50mm, preferably ranges from 5mm to 20mm, more preferably ranges from 8mm to 12mm, most preferably is 10mm.

13. Inhalation therapy device (1) according to any of the preceding claims 8 to 12, wherein the non-deflecting body is made of PTFE or PE.

14. Inhalation therapy device (1) according to any of the preceding claims 1 to 7, wherein the pressure-transmitting member (17, 18) is a flexible pressure-transmitting membrane that is impermeable to liquids and impermeable to gases and that is configured to transmit the pressure in the flow path (8) to the sensor (14) due to a deflection of the pressure-transmitting membrane caused by the pressure in the flow path (8) .

15. Inhalation therapy device (1) according to claim 14, wherein the diameter of the pressure-transmitting membrane ranges from 3mm to 50mm, preferably from 5mm to 20mm, more preferably from 6mm to 20mm, most preferably from 6mm to 15mm.

16. Inhalation therapy device (1) according to claim 14 or 15, wherein the thickness of the pressure-transmitting membrane ranges from 0.1mm to 2mm, preferably from 0.1mm to 1mm, more preferably from 0.2mm to 1mm, most preferably from 0.3mm to 0.5mm.

17. Inhalation therapy device (1) according to any of the preceding claims 14 to 16, wherein the pressure-transmitting membrane (17), (18) is made of elastomer, thermoplastic elastomer (TPE), latex, or silicone.

18. Inhalation therapy device (1) according to any of the preceding claims 14 to 17, wherein a volume between the pressure-transmitting membrane and the sensor (14) is larger than 1mm³, preferably larger than 5mm³, and is less than 1600mm³, preferably less than 1000mm³, more preferably less than 800 mm³.

19. Inhalation therapy device (1) according to any of the preceding claims 14 to 18, wherein the hardness of the pressure-transmitting membrane ranges from 10 to 100 shore A.

20. Inhalation therapy device (1) according to any of the preceding claims 1 to 7, wherein the pressure-transmitting member (17, 18) is a liquid- and gas impermeable gel that is configured to transmit the pressure in the flow path (8) to the sensor (14).
